# EUROPEAN PATENT APPLICATION

(11) **EP 4 474 699 A1**
(43) Date of publication of application: **11.12.2024**
(21) Application number: 24175214.6
(22) Date of filing: 10.05.2024
(51) Int. Cl.: F21V 33/00, G01D 11/24, G01L 19/06, G01N 33/00, F21V 21/28, F21W 131/205

(54) **OPERATING THEATRE LIGHT AND SYSTEM**

(30) Priority: 17.05.2023 GB 202307371
(71) Applicant: Brandon Medical Company Limited, Leeds West Yorkshire LS27 0EL (GB)
(72) Inventor: HALL, Adrian, Leeds, LS27 0EL (GB); PARTINGTON, Thomas, Leeds, LS27 0EL (GB); MILLER, Toby, Leeds, LS27 0EL (GB); ADAMS, David, Leeds, LS27 0EL (GB); STROUD, James, Leeds, LS27 0EL (GB)
(74) Representative: Murgitroyd & Company

(57) **Abstract**

An operating theatre light comprising at least one environmental sensor. The operating theatre light includes a sensor housing attached to the operating theatre light. The sensor housing defines an enclosure containing the at least one environmental sensor. The sensor housing is protected (e.g. sealed) against the ingress of liquid to the enclosure. The operating theatre light may have a transmitter or transceiver or wired connection for transmitting environmental sensor data produced by the at least one environmental sensor. An operating theatre system comprising the operating theatre light and a control panel comprising a receiver or transceiver configured to receive environmental sensor data transmitted by the operating theatre light.

## Description

### FIELD

This invention relates to an operating theatre light, to an operating theatre system including the operating theatre light.

### BACKGROUND

The environmental conditions within an operating theatre are known to have a bearing on patient outcomes. However, there are significant challenges to overcome to allow a meaningful assessment of environmental factors.

In general, in the past, it has not been possible to provide environmental sensors close to the patient and surgeon in an operating theatre, without requiring additional support structures that:
- may obscure the surgeon's view of the patient;
- obscure the surgical light;
- introduce unwanted clutter and trip hazards, and/or
- otherwise cause objections among surgeons and other medical professionals.

Surgical smoke detectors are available, but these require support structures and cabling. Other sensors, e.g., temperature or humidity, may be mounted on the operating theatre wall, but this does not give a true impression of the environment around the patient.

Most environmental sensors require access to free air and this is generally not compatible with operating room surface cleaning procedures. Ingress of cleaning (or bodily) fluids may represent an infection risk if the fluids were subsequently to drip onto a patient. The international standard for operating theatre lights (IEC 60601-2-41) describes a special procedure for assessing an Ingress Protection Rating that examines the potential for fluids to be retained and subsequently dripped during movement (e.g., rotation) of the theatre light. Environmental sensors that are used outdoors may utilise air inlets on the underside of their housing to prevent rainwater ingress. The use of downward facing air inlets to prevent liquid ingress are problematic in an operating theatre as they are generally facing the patient and may be exposed to streams of bodily fluids.

Individual environmental parameters may only give a partial explanation about why a particular patient outcome occurred. A suite of parameters may need to be monitored so that a correlation with patient outcome can be found. Additional factors may need to be considered, including the condition of critical equipment in the operating room. It is not currently possible to collect and communicate from multiple, concurrent data sources so that, for example, machine learning techniques can be used to optimise operating room conditions.

EP 2 636 964 B1 describes a method that involves arranging a measured value recorder in a portion of a wound area or of a standardized area such that an air quality parameter representing air quality is measured by the recorder. The parameter is measured in an area of operating lamps present in an operating room. The air to be supplied into the operating room is regulated with regards to speed and/or amount or volume as a function of the measured air quality parameter. A corresponding value is utilized as a correcting variable for regulating or controlling the air to be supplied to the operating room. The parameter is selected from a group consisting of air speed, turbulence, temperature, particle concentration, particle number with and/or without germ load.

EP 2 995 875 B1 describes a process for operating a clean room and a control device for a clean room, the clean room comprising a work room, a room ventilation system and a control device, the room ventilation system being controlled and/or regulated by means of the control device, an air exchange rate in the work room and a pressure difference between the work room and an environment being generated by means of the room ventilation system, the control device comprising at least one sensor device for registering an actual value representing an operating parameter, wherein the air exchange rate is adjusted by means of the control device in such a manner that the actual value is in the range of a target value.

### SUMMARY

Aspects of the present disclosure are set out in the accompanying independent and dependent claims. Combinations of features from the dependent claims may be combined with features of the independent claims as appropriate and not merely as explicitly set out in the claims.

According to an aspect of the invention, there is provided an operating theatre light comprising:
at least one environmental sensor; and
a sensor housing attached to the operating theatre light,
wherein the sensor housing defines an enclosure containing the at least one environmental sensor,
wherein the sensor housing is protected against the ingress of liquid to the enclosure, and
wherein the sensor housing includes at least one inlet vent and at least one outlet vent to allow the ingress and egress of air and/or particulates to and from the enclosure while preventing the ingress and egress liquid to and from the enclosure.

Placement of at least one environmental sensor in/on an operating theatre light allows the environmental sensor(s) to be situated close to the surgeon/patient in a manner that does not adversely interfere with or clutter the operating theatre.

The protection (e.g., sealing) of the sensor housing against the ingress of liquid to the enclosure can prevent damage to the environmental sensor(s) which may otherwise be caused by their coming into contact with liquids such as disinfectant cleaning liquids, blood or any other liquids. This can also allow the sensor(s) to be provided in a manner that does not allow liquids to be retained within the enclosure that may subsequently drip onto the patient.

The sensor housing may include at least one inlet vent and at least one outlet vent to allow the ingress and egress of air and/or particulates (such as smoke particles) to and from the enclosure while preventing the ingress and egress liquid to and from the enclosure. This can allow sensors (e.g., particulate sensors, humidity sensors) that require access to the air surrounding the patient/surgeon to acquire such access, in a manner that prevents liquid from entering the enclosure.

The at least one inlet vent and/or the at least one outlet vent may include an aperture passing through the sensor housing. The at least one inlet vent and/or the at least one outlet vent may also include a gas permeable, particulate (e.g., smoke) permeable, liquid impermeable, membrane covering the aperture. Again, this can allow sensors (e.g., particulate sensors, humidity sensors) that require access to the air surrounding the patient/surgeon to acquire such access, in a manner that prevents liquid from entering the enclosure.

The at least one inlet vent and/or the at least one outlet vent may further include a frustoconical recess defining the aperture. In embodiments in which the aforementioned membrane is provided, the membrane may cover the aperture at a narrowest part of the recess. The recessed position of the membrane may assist in preventing the membrane from coming into contact with liquid running along the surface of the sensor housing.

The at least one inlet vent and/or the at least one outlet vent may further include an arm extending across the recess at its widest point, to provide a path across the recess for liquid to flow over the recess without entering the recess. This can allow liquid running along the surface of the enclosure to be diverted past the recess and, in particular, the membrane.

A part of the sensor housing including the at least one inlet vent and/or the at least one outlet vent may be removable to gain access to the at least one sensor in the enclosure. This can allow for servicing/replacement of the sensors and may also allow checks to be performed to verify the absence of liquids within the enclosure.

The operating theatre light may also include a fan or pump for drawing air and/or particulates into the enclosure through the at least one inlet vent. This may enhance the ability of certain sensors to evaluate the air surrounding the patient.

The at least one environmental sensor may include:
a temperature sensor;
a humidity sensor;
an accelerometer;
a light sensor;
a particulate sensor; and/or
a sound sensor.

Further examples of environmental sensor(s) which may be present in the sensor unit include:
gas sensors;
volatile organic compound (VOC) sensors;
airborne biological matter sensors;
pressure sensors;
motion sensors;
pH sensors;
proximity sensors; and/or
position sensors.

The gas sensor may, for instance be a medical gas sensor. The gas sensor may be configured to detect, for instance, carbon dioxide, carbon monoxide, anaesthetic gas, formaldehyde, oxygen, or nitrous oxide sensor.

The sound sensor may, for instance, be a noise detector which can be used to monitor noise levels (e.g., associated with air conditioning, talking, surgical tool usage and so forth) in the operating theatre.

Other environmental sensor types are envisaged.

The operating theatre light may include a temperature sensor and at least one other environmental sensor. The operating theatre light may further include a pathway located in the enclosure. The pathway may extend between the inlet vent to the outlet vent. The temperature sensor may be located at a position on the pathway that is closer to the inlet vent than the at least one other environmental sensor. This arrangement can allow sensors that may affect the temperature of the incoming air and/or particulates to be placed after temperature sensor, so as to make the temperature sensor readings more accurate.

The operating theatre light may also include a light housing and an articulated arm having a first end and a second end. The first end may be configured to be attached to a surface of an operating theatre. The second end may be attached to the light housing. The sensor housing may be attached to the articulated arm at a location intermediate the first end and the second end. This can allow the environmental sensor(s) to be located in the vicinity of the patient/surgeon in a manner that does not complicate the design of the theatre lights themselves, and/or in a manner that does not cause the proximity of the sensors too close to the lights to distort the sensor readings (e.g., temperature). Providing the environmental sensor(s) in a separate sensor unit as opposed to in the light housing also has the benefit that a high degree of ingress protection can be obtained while also ensuring that the environmental sensor(s) have appropriate access to the air in the vicinity of the patient.

The operating theatre light may further include a transmitter or transceiver or wired connection for transmitting environmental sensor data produced by the at least one environmental sensor. This can allow the environmental sensor data to be passed on for further use outside the operating theatre light itself. These uses may include display of the sensor data for reference by the operating theatre occupants, the generation of actions to be performed by, for instance an air conditioning system, ventilation system and/or smoke extraction system in response to the sensor data, storage of the sensor data for later reference and/or analysis of the sensor data, e.g., by an artificial intelligence or machine learning tool (e.g., to correlate environmental conditions in the operating theatre with patient outcomes).

According to another aspect of the invention, there is provided an operating theatre system comprising:
an operating theatre light of the kind set out above; and
a control panel comprising a receiver or transceiver or wired connection configured to receive environmental sensor data transmitted by the operating theatre light.

As noted below, the control panel may also be configured to re-transmit the environmental sensor data transmitted by the operating theatre light. The control panel of the system may allow for control of the environmental sensors and/or the lights of the light housing.

The control panel may include a display. The control panel may be configured to display information relating to the received environmental sensor data using the display. This can allow occupants of the operating theatre to monitor the environmental conditions surrounding the patient. The control panel may also include a computer system comprising, for instance, a processor, memory, storage and I/O components.

The control panel may be configured to communicate information relating to the received environmental data via a communications network to at least one of:
a building management system;
a digital processing system;
a digital storage system;
an air conditioning system;
a ventilation system;
a smoke extraction system;
a cloud server.

This can allow some of the usages of the sensor data noted above to be implemented.

According to a further aspect of the invention, there is provided an operating theatre light comprising at least one environmental sensor.

### BRIEF DESCRIPTION OF THE DRAWINGS

Embodiments of this disclosure will be described hereinafter, by way of example only, with reference to the accompanying drawings in which like reference signs relate to like elements and in which:
Figure 1A shows an operating theatre light according to an embodiment of this disclosure;
Figure 1B shows another view of the operating theatre light of Figure 1, according to an embodiment of this disclosure;
Figure 2 shows a side view of the operating theatre light of Figure 1, according to an embodiment of this disclosure;
Figure 3 shows a sensor unit according to an embodiment of this disclosure;
Figure 4 shows a close-up view of part of the sensor unit of Figure 3, according to an embodiment of this disclosure;
Figure 5 shows a front side view of the sensor unit of Figure 3, according to an embodiment of this disclosure;
Figure 6 shows a side view of the sensor unit of Figure 3, according to an embodiment of this disclosure;
Figure 7A shows a close-up view of part of the front of the sensor unit of Figure 3, according to an embodiment of this disclosure;
Figure 7B shows a close-up view of part of the side of the sensor unit of Figure 3, according to an embodiment of this disclosure;
Figure 8A shows a close-up view of one of the inlet/outlet vents of the sensor unit, according to an embodiment of this disclosure;
Figures 8B, 8C and 8D show various section views of the inlet/outlet vent shown in Figure 8A;
Figure 9 shows the sensor housing of Figure 3 with the sensor unit cover removed, according to an embodiment of this disclosure;
Figure 10 shows a schematic of an operating theatre environment according to an embodiment of this disclosure;
Figure 11 shows a schematic of an operating theatre environment according to an embodiment of this disclosure; and
Figure 12 shows a communications network according to an embodiment of this disclosure.

### DETAILED DESCRIPTION

Embodiments of this disclosure are described in the following with reference to the accompanying drawings.

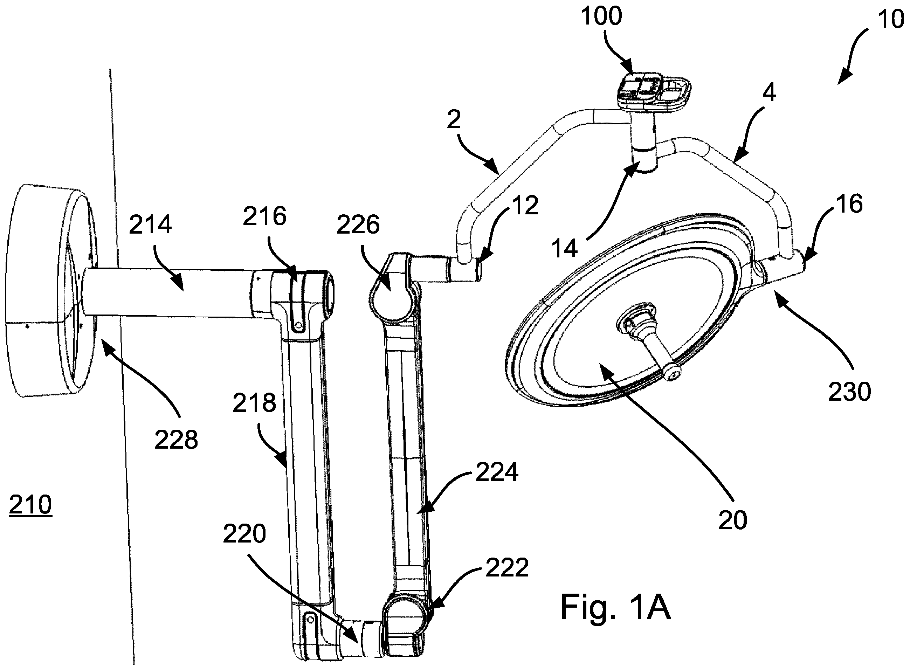

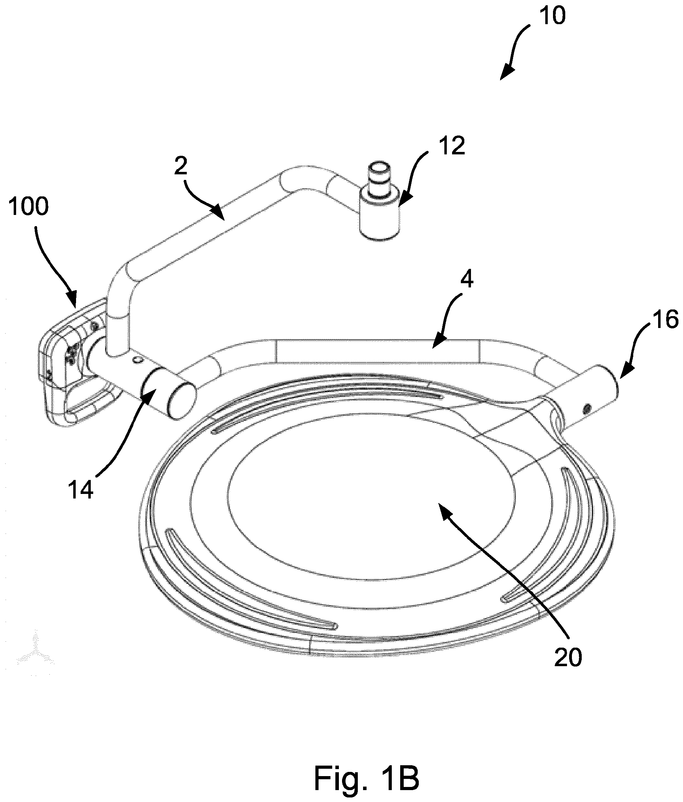

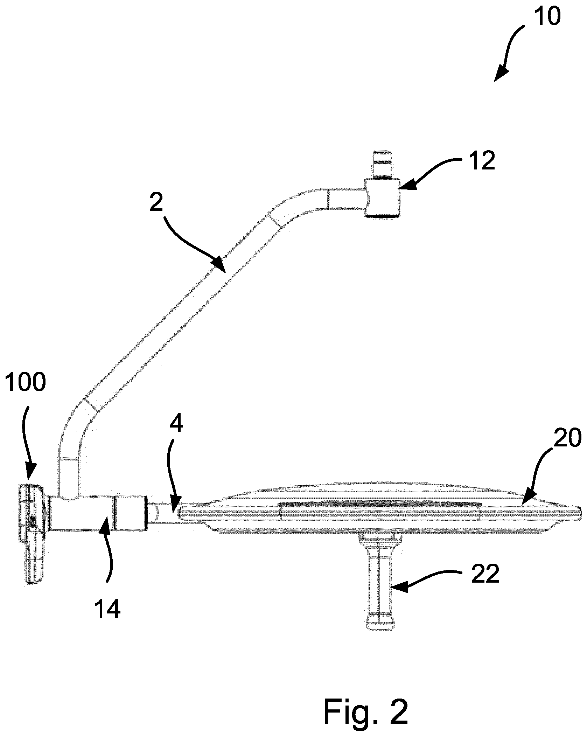

Figures 1A, 1B and 2 show different views of an operating theatre light 10 according to an embodiment of this disclosure. As will be described in more detail below, the operating theatre light 10 includes at least one environmental sensor.

In this embodiment, the operating theatre light 10 includes a light housing 20 and an articulated arm. The articulated arm may have a first end 228 and a second end 230. The first end 228 may be attachable to a surface 210 (ceiling, wall...) of an operating theatre. The second end 230, which may generally be opposite the first end, may be attached to the light housing 20. The articulated arm in this embodiment includes a number of arm sections 2, 4, 214, 218, 224. The arm sections 2, 4 collectively form a cardanic/yoke section of the articulated arm. The cardanic/yoke section of the articulated arm may be located at or toward an end of the articulated arm that is distal the first end 228 of the articulated arm. While the cardanic/yoke section of the articulated arm shown in Figures 1A, 1B and 2 has two arm sections 2, 4, it is envisaged that only a single arm section, or indeed more than two arm sections, may be provided. Similarly, while the articulated arm shown in Figures 1A, 1B and 2 has five arm sections 2, 4, 214, 218, 224 it is envisaged that only a single arm section, or indeed more than five arm sections, may be provided. The arm sections 2, 4, 214, 218, 224 are joined together at their ends by pivotable joints 14, 216, 220, 222, 226. Pivotable joints 12, 16 may also be provided at the first and second ends of the of the cardanic/yoke section of the articulated arm. The various joints 12, 14, 16, 216, 220, 222, 226 can allow the articulated arm to be manipulated for placing the light housing 20 in a desired position, with a desired orientation. As shown in Figure 2, the operating theatre light 10 may further include a handle 22, for assisting in these manipulations.

The light housing 20 includes one or more lights. These lights may, for instance, comprise Light Emitting Diodes (LEDs), although it is also envisaged that other kinds of conventional lights may be used. Cabling for providing power to the lights of the light housing 20 may pass through the articulated arm, for connection at the first end.

As described herein, the operating theatre light 10 includes at least one environmental sensor. It is envisaged that the sensor(s) may be located anywhere on the operating theatre light 10. Placement of the at least one environmental sensor in/on an operating theatre light 10 allows the environmental sensor(s) to be situated close to the surgeon/patient in a manner that does not adversely interfere with or clutter the operating theatre.

In the present embodiment, the at least one environmental sensor is located within a sensor housing 102 of a sensor unit 100. The sensor unit 102 may located part way along the articulated arm, such as at one of the joins between two of the arm sections. This kind of positioning of the sensor unit 100 can allow the at least one environmental sensor to be located in the vicinity of the patient/surgeon in a manner that does not complicate the design of the theatre lights themselves (e.g., the design of the light housing 20). This kind of positioning of the sensor unit 100 also can allow the at least one environmental sensor to be located in the vicinity of the patient/surgeon in a manner that does not cause the proximity of the sensors too close to the lights to distort the sensor readings (e.g., temperature).

In some embodiments, the sensor unit 102 may be located part way along the cardanic/yoke section of the articulated arm. In particular, in the embodiment of Figures 1A, 1B and 2, the sensor unit 100 is attached to the joint 14 between the two arm sections 2, 4.

In some embodiments, the sensor unit 100 can also include external controls (buttons, dials, switches etc.) for controlling the lights of the operating theatre light (e.g., brightness, colour temperature, etc.).

In some embodiments, the sensor unit 100 may include a computer system comprising, for instance, a processor, memory, storage and I/O components. The computer system may be configured to process (e.g., pre-process) the sensor data described herein and/or to receive and process signals such as control signals from the external control buttons described above and/or from the control panel 200, building management or air conditioning systems to be described below.

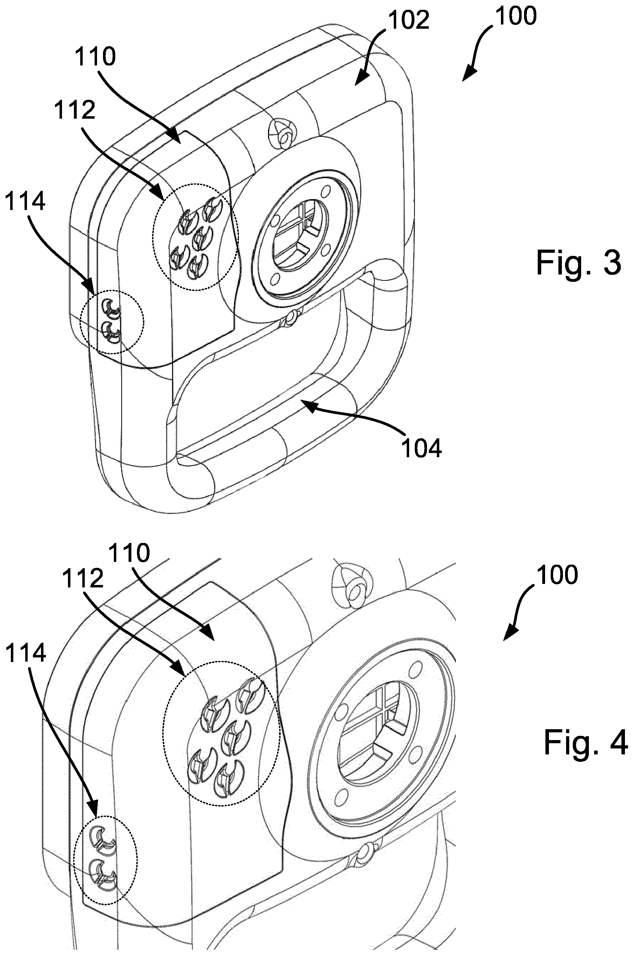

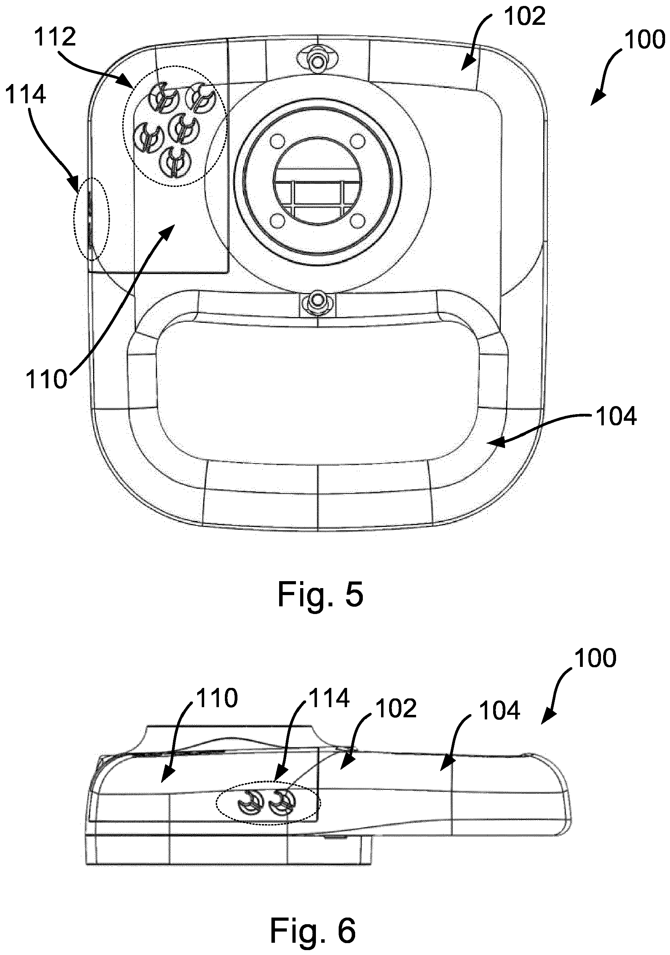

Figures 3 to 9 show various views of the sensor unit 100. The sensor housing 102 of the sensor unit 100 in this embodiment defines an enclosure 170 containing the at least one environmental sensor. The sensor housing 100 may generally be protected (e.g., sealed) against the ingress of liquids (e.g., such as disinfectant cleaning liquids, blood or other liquids) to the enclosure. This can prevent damage to the environmental sensor(s), which may otherwise be caused by their coming into contact with liquids. This can also allow the sensor(s) to be provided in a manner that does not retain liquid (i.e., within the enclosure) that may subsequently drip onto the patient.

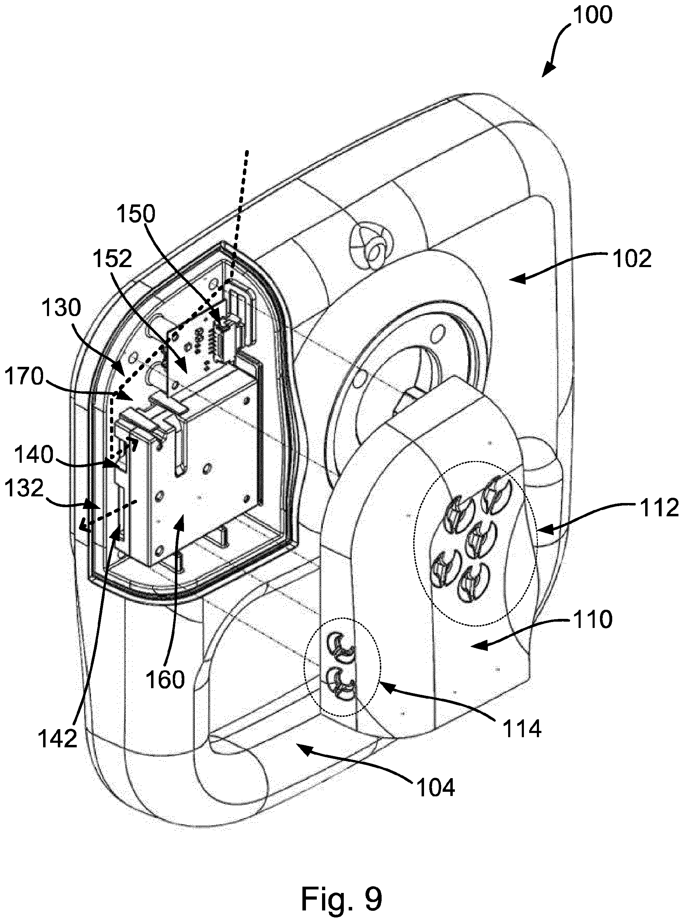

In some embodiments, the part of the enclosure containing the at least one environmental sensor may be part of a larger enclosure defined by the sensor housing 102. In such embodiments, the larger enclosure may also include other components (e.g., control electronics, power management components, transmitter/receiver/transceiver, wired connection(s) and such like) of the sensor unit 100. In other embodiments, as illustrated in Figure 9, the enclosure 170 may be separated off from the rest of the interior of the sensor unit 100 (which may contain other components of the sensor unit 100 as listed above), so as to prevent the rest of the interior of the sensor unit 100 from being exposed to the surrounding environment (e.g., the air in the airflow pathway 130/132 to be described below).

In some embodiments, as shown in Figure 9, the sensor housing 102 may include a removable sensor unit cover 110. This can allow access to be gained to the at least one sensor in the enclosure 170. This can allow for servicing/replacement of the sensors and may also allow checks to be performed to verify the absence of liquids within the enclosure. The sensor unit cover 110 may, in some embodiments, include the inlet vent(s) 112 and/or the outlet vent(s) 114 described below.

In some embodiments, the sensor housing 102 may be shaped to define a handle 104 of the sensor unit 100, to provide an additional option for manipulating the articulated arm for positioning the sensor unit 100 in relation to the light housing 20 and/or the patient/surgeon, and/or for adjusting the orientation of the sensor unit 100 upon the articulated arm.

The environmental sensor(s) may, according to embodiments of this invention, include, for instance a temperature sensor, a humidity sensor, a gas (e.g., medical gas) sensor, a light sensor, a pressure sensor, a pH sensor, a particulate (e.g. smoke) sensor, a sound sensor and/or any other kind of sensor for detecting aspects of the environment immediately surrounding the patient/surgeon in the operating theatre while a surgical procedure is taking place. The gas sensor may, for instance be a carbon dioxide, carbon monoxide, anaesthetic gas, formaldehyde, oxygen, or nitrous oxide sensor. The sound sensor may be for detecting noise levels in the operating theatre. In the embodiment shown in the Figures, the sensor unit 100 includes, for illustrative purposes, a temperature sensor 150 and a particulate sensor 160. Further examples of environmental sensor(s) which may be present in the sensor unit include:
volatile organic compound (VOC) sensors;
airborne biological matter sensors;
pressure sensors;
motion sensors (e.g., accelerometers);
proximity sensors; and/or
position sensors.

As will be described in more detail below, various uses of the sensor data collected by the environmental sensor(s) are envisaged.

In some embodiments, the sensor unit 100 may be provided with components that allow the sensor data collected by the environmental sensor(s) to be externally communicated. These components may include features such as a transmitter or transceiver, to allow the sensor unit 100 to transmit the sensor data over a wireless network (e.g., according to the IEEE 802.11 standard, although wireless communications protocols may also be used). Alternatively, the sensor unit 100 may be provided with a wired connection, which may extend through the articulated arm for coupling to a wired network or for onward connection to an external wireless transmitter or transceiver.

At least some of the sensors envisaged for inclusion in a sensor unit 100 according to embodiments of this invention require direct contact with air in the immediate vicinity of the surgeon/patient. According to embodiments, the sensor housing 102 is provided with one or more inlet vents 112 and one or more outlet vents 114 to allow air and/or particulates (such as smoke particles) to enter the enclosure 170 to be sampled by the environmental sensor(s) and subsequently exit the enclosure 170. It is envisaged that the number and size of the inlet vent(s) 112 and/or the outlet vent(s) 114 may be chosen to provide sufficient airflow within the enclosure 170 for the environmental sensors to operate correctly. In the present embodiment, five inlet vents 112 and two outlet vents 114 are provided, although it is envisaged that fewer or greater than this number may be provided as required. As will be described in more detail below, the inlet vent(s) 112 and the outlet vent(s) 114 may include features that assist in preventing the ingress and egress liquid to and from the enclosure 170, while nevertheless allowing the ingress and egress of air and particulates such as smoke particles.

In some embodiments, the sensor unit 100 may include a fan or a pump for drawing air and/or particulates into the enclosure through the inlet vent(s). This may enhance the ability of certain sensors to evaluate the air surrounding the patient/surgeon, since the sensor(s) need not rely upon air and/or particulates naturally passing into the enclosure 170 and a larger volume of air and/or particulates may thus be sampled.

In some embodiments, where more than one environmental sensor is provided in the enclosure 170, the positions of those sensors may be chosen (in relation to the position of the inlet vent(s) 112) such that air and/or particulates entering the enclosure 170 is incident upon certain ones of the environmental sensors before being incident on other ones of the environmental sensors. To implement this, an airflow pathway 130/132 may be defined within the enclosure 170, between the inlet vent(s) 112 and the outlet vent(s) 114. The ordering of the environmental sensors along the airflow pathway 130/132 may be chosen as noted above. This ordering can in particular be chosen as that sensors that may otherwise distort readings taken by other sensors of the sensor unit can be located toward the end of the airflow pathway 130/132.

In the embodiment shown in Figure 9, for instance, a temperature sensor 150 is located at the beginning of the airflow pathway 130/132, while the particulate sensor 160 is located after the temperature sensor 150 in the airflow pathway 130/132. This arrangement can prevent any changes in the temperature of the air and/or particulates associated with heat produced by internal components of the particulate sensor 160 (or indeed any other components within the enclosure 170) from producing distorted temperature readings at the temperature sensor 150 since, in the arrangement shown, the temperature of the air and/or particulates is read by the temperature sensor 150 almost immediately after the air and/or particulates has entered the enclosure 170 through the inlet vent(s) 112. As mentioned above, it will be appreciated that analogous considerations may apply to other sensor types and their location along the airflow pathway 130/132 may be chosen accordingly.

It is noted that some sensors in the airflow pathway 103/132 may require their own inlets and outlets for sampling the air and/or particulates (e.g., see the inlet 140 and out 142 of the particulate sensor 160 shown in Figure 9), while other sensors may require only that they are placed within the airflow pathway 103/132 so that the airflow is incident upon them (e.g., see the temperature sensor 150 shown in Figure 9).

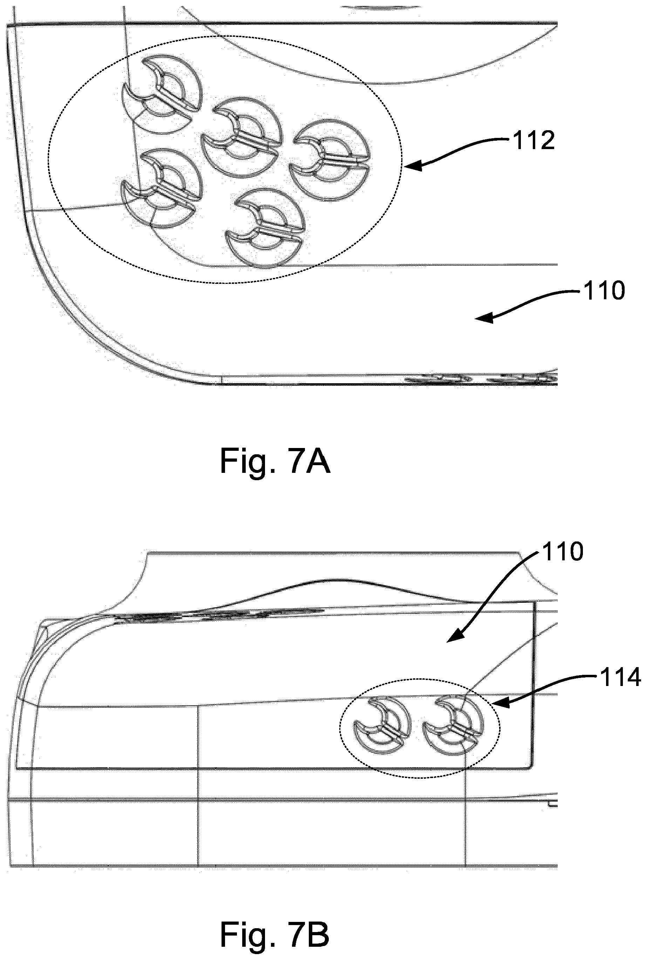

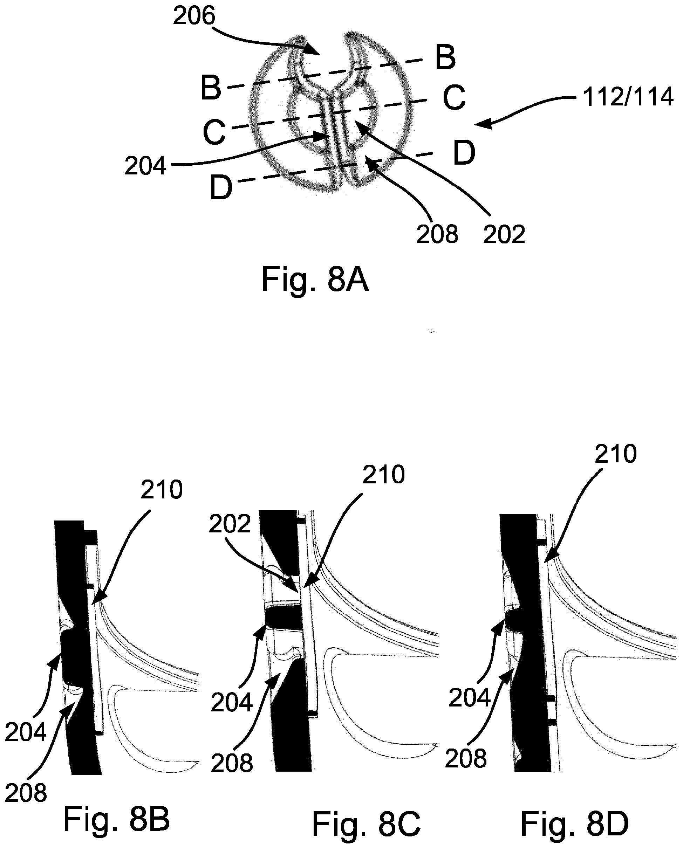

As noted previously, the inlet vent(s) 112 and/or the outlet vent(s) 114 may include features may include features that assist in preventing the ingress and egress liquid to and from the enclosure 170. Features of this kind will now be described with reference to the views of the inlet vent(s) 112 and/or the outlet vent(s) 114 shown in Figures 7A, 7B and 8A to 8D, respectively. Figures 8B to 8D each show a cross section of the inlet/outlet vent 112/114 shown in Figure 8A, through the lines BB, CC and DD, respectively. Note that in the present embodiment, the inlet vent(s) 112 and the outlet vent(s) 114 have substantially the same shape and configuration, although this is not considered to be essential.

In this embodiment, each inlet vent 112 and each outlet vent 114 includes an aperture 202 that passes through the sensor housing 102. To inhibit the ingress of liquid to the enclosure 170 through the aperture 202, while still allowing air, smoke and such like to enter, a gas permeable, particulate (e.g., smoke) permeable, liquid impermeable, membrane 210 may also be provided, which covers the aperture. In this embodiment, the membrane 210 is located on an interior surface of the sensor 102, so as to cover the aperture 202, although other configurations may be used. The membrane 210 may, in some embodiments, be hydrophobic. The membrane may, for instance, comprise a gas permeable, particulate (e.g., smoke) permeable, Polytetrafluoroethylene (PTFE) material.

In some embodiments, the aperture 202 may include a substantially frustoconical recess 208, with its widest part at the outer surface of the enclosure. As shown in Figure 8, the recess 208 may lead into a steeper, narrower section of the aperture 202. This narrowest part of the aperture 202 may be covered by the membrane 210. The recessed position of the membrane 210, away from the outmost surface of the sensor housing 102 in this manner may assist in preventing the membrane from coming into contact with liquid running along the surface of the sensor housing 102.

In some embodiments, the inlet vent(s) 112 and/or the outlet vent(s) 114 further include an arm 204 that extends across the aperture 202 at its widest point (e.g., substantially flush with the outermost surface of the sensor housing 102). The arm 204 can provide a path across the recess 208 and/or aperture 202 for liquid to flow over the recess 208 and/or aperture 202 without entering the recess 208 and/or aperture 202 and possibly coming into contact with the membrane 210. In some embodiments, the arm 204 may have a funnel section 206 at one end. The funnel section 206, which may be substantially flat and flush with the outer surface of the sensor housing 102 may form a widened, open section at one end of the arm 204 for collecting fluid and then channelling it to, and thus across, the arm 204. In this way, the ability of the arm 204 to prevent fluid from entering the recess 208 and/or aperture 202 may be enhanced. Note that in some embodiments, an underside of the arm 204 may be in contact with the membrane 210 (e.g., see Figures 8B, 8C and 8D), although this is not essential. Note also that the arm 204 may act to section off the aperture 202 into two or more separate sub-apertures. For instance, in the embodiment of Figure 8, the arm 204 divides the aperture into two, smaller, sub-apertures.

A further advantage of the design of the inlet vent(s) 112 and/or the outlet vent(s) 114 is that they can allow for easy cleaning. In particular, the inlet vent(s) 112 and/or the outlet vent(s) 114 are open enough to allow a cloth or other cleaning implement to enter the recess 208 for gaining access to the aperture 202. The inlet vent(s) 112 and/or the outlet vent(s) 114 therefore do not include any parts that cannot easily be accessed.

Accordingly, embodiments of this invention can provide for the location of environmental sensors in the vicinity of the surgeon/patient during a surgical procedure, while also taking steps to minimise the risk of liquid (e.g., blood or disinfectant cleaning liquids) that has splashed onto the sensor unit 100 entering the enclosure 170 and contacting the sensors themselves, or subsequently dripping back onto the patient or a future patient.

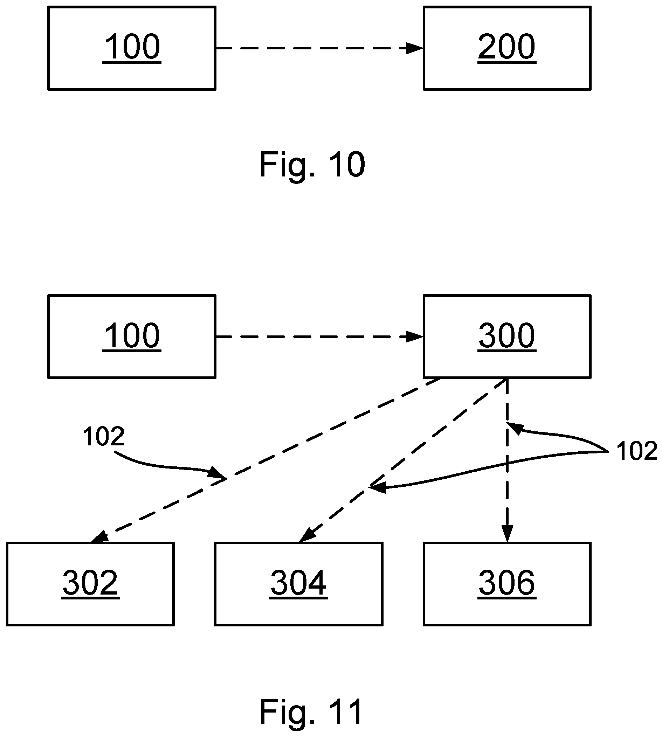

As noted previously, a number of uses for the data collected by the environmental sensors are envisaged. Figures 10 and 11 each show a schematic of an operating theatre environment according to an embodiment of this disclosure.

In Figure 10, the sensor unit 100 is shown as being connected to a control panel 200. The control panel 200 may, for instance, be attached to a surface (e.g., a wall) of the operating theatre for easy access and viewing by occupants of the operating theatre. As noted above, the sensor unit 100 may include features implementing the wired or wireless transmission of the data collected by the environmental sensors. The control panel 200 may similarly include features (e.g., a receiver/transceiver or a wired connection) for implementing the wired or wireless reception of the data.

The control panel 200 may include a display. This may allow the control panel 200 to show information relating to the data collected by the environmental sensor of the operating theatre light 10 (in some embodiments, a simple display panel/monitor may be used to display the data locally, without including the control features of the control panel). Such information may simply include the raw data, or alternatively the data may be processed in some way to improve the clarity with which the information is presented (e.g., by placing the data into graphs, histograms or the like). The presented information may assist occupants of the operating theatre in monitoring the environmental conditions in the operating theatre. In some embodiments, alarms (e.g., audible, visual...) may be issued by the control panel if any of the measured data fall outside predetermined acceptable thresholds. By way of example only, these alarms may warn the occupants of the operating theatre if the environmental conditions have become too hot/cold, humid/dry, smoky, etc.). The control panel 200 may also include a computer system as noted above.

The control panel may also be used to send control signals to the sensor unit 100, e.g., for powering the sensor unit on and off, for initialising the sensor(s), and/or for configuring the sensor(s).

The embodiment in Figure 11 is similar to the embodiment in Figure 10, with the exception that in the embodiment of Figure 11, control panel 300 is configured to communicate information relating to the received environmental data via a communications network. The communications network may be a wired and/or wireless communications network. This can allow the data to be sent to at least one of the following recipients: a digital storage system, an air conditioning system, a ventilation system, a smoke extraction system, a cloud server and a Building Management System. For instance, in Figure 11, it is shown that information or control signals relating to the data produced by the particulate sensor 160 may be forwarded by the control panel to a ventilation system 304 and/or a smoke extraction system 306, whereas information relating to the temperature data collected by the temperature sensor 150 and/or a humidity sensor may be sent to an air conditioning system 302. The information may comprise the actual data as collected or may comprise a preprocessed version of the data. In some embodiments, the information may comprise control signals for controlling the ventilation system 304, the smoke extraction system 306 and/or the air conditioning system 302 according to requirements determined by algorithms located in the control panel or computer system using the collected data, or according to requests inputted to the control panel by an occupant of the operating theatre.

While Figure 11 has been described in the context of a control panel that acts as a stopping point for the data, which sits between the operating theatre light and the ventilation system 304, the smoke extraction system 306 and/or the air conditioning system 302, it is also envisaged that the data from the operating theatre light 10 may be sent directly to the ventilation system 304, the smoke extraction system 306 and/or the air conditioning system 302, or to a control system located outside the operating theatre.

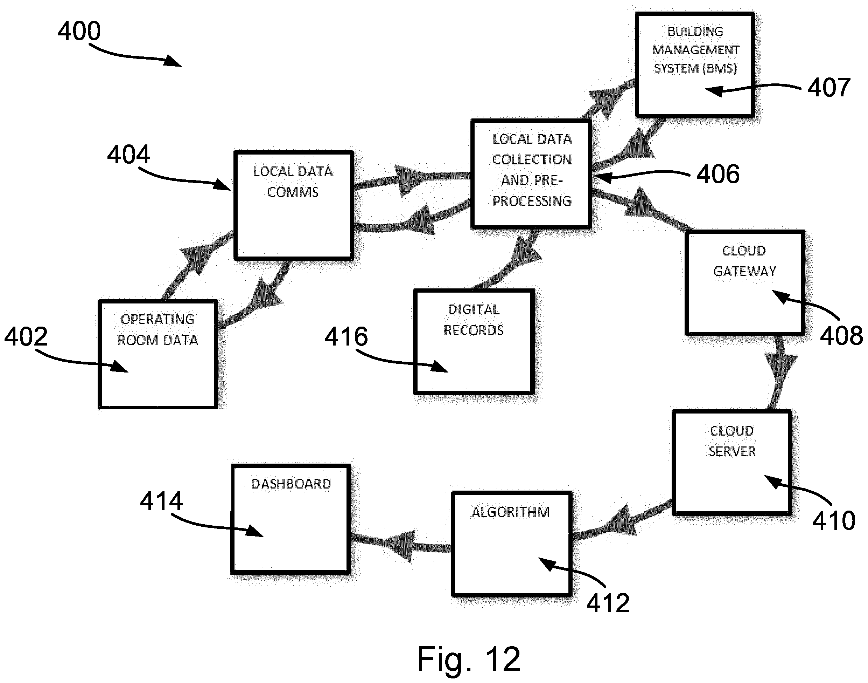

Figure 12 shows a communications network 400 according to an embodiment of this disclosure, and how the data 402 collected by the environmental sensor(s) of the operating theatre light may be distributed among other components connected to the network.

As described above, the operating theatre light and control panel may be provided with components for communicating the data between them. These components may include transmitters/transceivers/receivers wireless communications and/or a wired connection, which are collectively indicated in Figure 12 using reference numeral 404.

In block 406, local data collection and pre-processing of the data may be implemented by components of the operating theatre light and/or the control panel as noted previously.

Cloud gateway 408 may be used to pass the collected data either directly or indirectly (e.g., via the control panel) onto the cloud, for subsequent onward receipt at further components such as data storage 416 or a cloud server 410. In some embodiments, the data may be passed directly to another entity (i.e., not via the cloud gateway 408). For instance, in the example of Figure 12, the data may be passed directly from block 406 to a building management system 407.

In some embodiments, algorithms 412 may be used to analyse the data. Such analysis may involve feeding the data to an artificial intelligence or machine learning tool (e.g., to correlate environmental conditions in the operating theatre with patient outcomes). The outcome of this analysis may be fed back to surgical planning tools or a dashboard 414 to allow the environmental conditions present in future procedures to be optimised in line with any observed correlations between the environmental conditions and positive patient outcomes.

Accordingly, there has been described an operating theatre light comprising at least one environmental sensor. The operating theatre light includes a sensor housing attached to the operating theatre light. The sensor housing defines an enclosure containing the at least one environmental sensor. The sensor housing is protected (e.g. sealed) against the ingress of liquid to the enclosure. The operating theatre light may have a transmitter or transceiver or wired connection for transmitting environmental sensor data produced by the at least one environmental sensor. An operating theatre system comprising the operating theatre light and a control panel comprising a receiver or transceiver configured to receive environmental sensor data transmitted by the operating theatre light.

Although particular embodiments of this disclosure have been described, it will be appreciated that many modifications/additions and/or substitutions may be made within the scope of the claims.

## Claims

1. An operating theatre light comprising:
at least one environmental sensor; and
a sensor housing attached to the operating theatre light,
wherein the sensor housing defines an enclosure containing the at least one environmental sensor,
wherein the sensor housing is protected against the ingress of liquid to the enclosure, and
wherein the sensor housing includes at least one inlet vent and at least one outlet vent to allow the ingress and egress of air and/or particulates to and from the enclosure while preventing the ingress and egress liquid to and from the enclosure.

2. The operating theatre light of claim 1, wherein the at least one inlet vent and/or the at least one outlet vent comprises an aperture passing through the sensor housing.

3. The operating theatre light of claim 2, wherein the at least one inlet vent and/or the at least one outlet vent further comprises a gas permeable, particulate permeable, liquid impermeable, membrane covering the aperture.

4. The operating theatre light of claim 2 or claim 3, wherein the at least one inlet vent and/or the at least one outlet vent comprises a frustoconical recess defining the aperture.

5. The operating theatre light of claim 4 when dependent on claim 3, wherein the membrane covers the aperture at a narrowest part of the recess.

6. The operating theatre light of claim 4 or claim 5, wherein the at least one inlet vent and/or the at least one outlet vent further comprises:
an arm extending across the recess at its widest point, to provide a path across the recess for liquid to flow over the recess without entering the recess.

7. The operating theatre light of any preceding claim, wherein a part of the sensor housing including the at least one inlet vent and/or the at least one outlet vent is removable to gain access to the at least one sensor in the enclosure.

8. The operating theatre light of any preceding claim, comprising a fan or pump for drawing air and/or particulates into the enclosure through the at least one inlet vent.

9. The operating theatre light of any preceding claim, wherein the at least one environmental sensor comprises:
a temperature sensor;
a humidity sensor;
an accelerometer;
a light sensor;
a particulate sensor; and/or
a sound sensor.

10. The operating theatre light of any preceding claim, wherein the operating theatre light includes a temperature sensor and at least one other environmental sensor, wherein the operating theatre light further comprises:
a pathway located in the enclosure, wherein the pathway extends between the inlet vent to the outlet vent, wherein the temperature sensor is located at a position on the pathway that is closer to the inlet vent than the at least one other environmental sensor.

11. The operating theatre light of any preceding claim, wherein the operating theatre light comprises:
a light housing; and
an articulated arm having a first end and a second end,
wherein the first end is configured to be attached to a surface of an operating theatre,
wherein the second end is attached to the light housing, and
wherein the sensor housing is attached to the articulated arm at a location intermediate the first end and the second end.

12. The operating theatre light of any preceding claim, further comprising a transmitter or transceiver or wired connection for transmitting environmental sensor data produced by the at least one environmental sensor.

13. An operating theatre system comprising:
an operating theatre light according to claim 12; and
a control panel comprising a receiver or transceiver or wired connection configured to receive environmental sensor data transmitted by the operating theatre light.

14. The operating theatre system of claim 13, wherein the control panel includes a display, and wherein the control panel is configured to display information relating to the received environmental sensor data using the display.

15. The operating theatre system of claim 13 or claim 14, wherein the control panel is configured to communicate information relating to the received environmental data via a communications network to at least one of:
a building management system;
a digital storage system;
an air conditioning system;
a ventilation system;
a smoke extraction system;
a cloud server.
